# EUROPEAN PATENT SPECIFICATION

(11) **EP 0 735 900 B1**
(45) Date of publication and mention of the grant of the patent: **05.06.2002**
(21) Application number: 95903299.6
(22) Date of filing: 28.11.1994
(51) Int. Cl.: A61K 49/00, A61P 43/00

(54) **PARAMAGNETIC DIAGNOSTIC FORMULATIONS AND THEIR METHOD OF USE**
PARAMAGNETISCHE DIAGNOSTISCHE ZUSAMMENSETZUNGEN UND VERFAHREN ZUR VERWENDUNG
FORMULATIONS PARAMAGNETIQUES DE DIAGNOSTIC ET METHODES POUR LEUR UTILISATION

(30) Priority: 24.12.1993 IT MI932728
(43) Date of publication of application: 09.10.1996
(73) Proprietor: BRACCO S.p.A., 20134 Milano (IT); Bracco Imaging S.p.A., 20134 Milano (IT)
(72) Inventor: AIME, Silvio, I-10041 Carignano (IT); BOTTA, Mario, I-12030 Villanovetta (IT); CALABI, Luisella, I-20133 Milano (IT); PALEARI, Lino, I-20035 Lissone (IT)
(74) Representative: Minoja, Fabrizio, Dr.
(86) International application number: EP9403944
(87) International publication number: WO9517910

(56) References cited:
- EP-A- 0 095 124
- EP-A- 0 122 000
- EP-A- 0 135 125
- WO-A-94/27977
- WO-A-94/27978
- US-A- 4 915 933
- DATABASE CHEMABS CHEMICAL ABSTRACTS SERVICE, COLUMBUS, OHIO, US AN=118:55405, INGWALL, JOANNE S. 'Measuring cation movements across the cell wall using NMR spectroscopy: sodium movements in striated muscle' & NMR (1992), 28(IN-VIVO MAGN. RESON. SPECTROSC. III), 131-60 CODEN: NBPPD3;ISSN: 0170-5989, 1992
- DATABASE CHEMABS CHEMICAL ABSTRACTS SERVICE, COLUMBUS, OHIO, US AN=118:92999, HUTCHISON, ROBERT B. ET AL 'Suppression of sodium nuclear magnetic resonance double-quantum coherence by chemical shift and relaxation reagents' & J. CHEM. PHYS. (1992), 97(12), 8934-40 CODEN: JCPSA6;ISSN: 0021-9606, 1992
- DATABASE CHEMABS CHEMICAL ABSTRACTS SERVICE, COLUMBUS, OHIO, US AN=116:169116, KOHLER, SUSAN J. ET AL 'In vivo sodium chemical shift imaging' & MAGN. RESON. MED. (1992), 23(1), 77-88 CODEN: MRMEEN;ISSN: 0740-3194, 1992
- DATABASE MEDLINE US NATIONAL LIBRARY OF MEDICINE (NLM), BETHESDA, MD, US AN=92382454, & MAGN. RESON. MED., vol. 26, no. 2, August 1992 pages 308-312, BHUJWALLA Z.M. ET AL. 'SPATIAL HETEROGENICITY OF THE METABOLIC RESPONSE OF RIF-1 TUMORS TO A AVASOACTIVE AGENT EVALUATED IN VIVO BY ONE-DIMENSIONAL 31P CHEMICAL SHIFT IMAGING.'
- DATABASE MEDLINE US NATIONAL LIBRARY OF MEDICINE (NLM), BETHESDA, MD, US AN=92008702, & EUR. J. VASC. SURG., vol. 5, no. 4, August 1991 pages 383-396, MOHIADDIN R.H. 'm.r. orphological chemical shift imaging in peripheral vascular disease.'
- DATABASE MEDLINE US NATIONAL LIBRARY OF MEDICINE (NLM), BETHESDA, MD, US an=92063380, & BR. J. RADIOL., vol. 64, no. 766, October 1991 pages 923-928, SMITH S. R. ET AL. 'TUMOUR PH AND RESPONSE TO CHEMOTHERAPY: AN IN VIVO 31P MR SPECTROSCOPY STUDY IN NON-HODGKIN'S LYMPHOMA.'

## Description

This invention refers to the use according to claim 1 for the preparation of an MRI contrast agent for a method for Magnetic Resonance Imaging (MRI) using proton signals of paramagnetic metal-ion complexes, based on the use of Chemical Shift Imaging (CSI) techniques, as well as Magnetic Resonance Spectroscopy (MRS) for the control and recording of biological parameters.

The use in medicine of a high number of these complexes is widely reported: for instance as stabilizers for the pharmaceutical preparations or antidotes in case of ingestion of toxic metal species.

Physiologically tolerable complexes formed by chelating agents and bi- or trivalent metal ions are used as diagnostic agents in imaging techniques such as X-ray, nuclear magnetic resonance (NMR) and scintigraphy.

In particular, magnetic resonance imaging (MRI) is a renowned powerful diagnostic procedure used in medical practice (see Stark, D.D., Bradley, W. G., Jr., Eds. "Magnetic Resonance Imaging" The C. V. Mosby Company, St. Louis, Missouri (USA), 1988) which relies on the use of paramagnetic pharmaceutical compositions, preferably containing chelated complexes of bi- or trivalent paramagnetic metal ions, usually belonging to the class of transition metals, or rare earth, with aminopolycarboxylic acids and/or their derivatives or analogues.

The images (basically coming from the NMR signal of water protons) are the result of a complex interaction of different parameters, such as proton density and T₁ and T₂ relaxation times. A contrast enhancement can be obtained through the administration of exogenous chemical substances which significantly change the resonance properties of nearby water protons (see Lauffer, R.B. Chem. Rev. 1987,87,901).

Paramagnetic contrast media used in MRI modify the relaxation times of water protons in tissues, in which said contrast medium concentrates, and therefore can enhance the differences among different tissues, or between healthy and pathological tissues.

Due to the high capacity of gadolinium complexes of reducing the relaxation times of hydrogen nuclei of nearby water molecules through dipolar interaction, scientists have investigated, patented and published some works on these complexes (see Lauffer). And some of them have been approved as MRI contrast media (Gd-DTPA-DImeg, N-methylglucamine salt of gadolinium diethylenetriaminepentaacetic acid, MAGNEVIST®, Schering; Gd-DOTA-DImeg, N-methylglucamine salt of gadolinium 1,4,7,10-tetraazacyclododecan-1,4,7,10-tetracetic acid, DOTAREM®, Guerbet).

A list of significant patent documents showing the state of the art in this diagnostic field, even though uncompleted, is represented by: EP 71564 (Schering), US 4639365 (Sherry), US-A-4615879 (Runge), DE-A-3401052 (Schering), EP 130934 (Schering), EP 65728 (Nycomed), EP 230893 (Bracco), US-A-4826673 (Mallinckrodt), US-A-4639365 (Sherry), EP 299795 (Nycomed), EP 258616 (Salutar), WO 8905802 (Bracco).

EP 95124 discloses in vivo NMR-measurement of temperature or pH by NMR-contrast agents using diamagnetic substances.

Chem. Abstr. 116:169116, EP-A-135125 and US-A-4915933 disclose imaging methods using paramagnetic complexes which, when applied in vivo shift the resonance of selected endogenous species to be detected.

WO 94/27977 and WO 94/27978, published after the priority date, disclose macrocyclic paramagnetic metal complexes which may be used as chemical shift contrast agents.

Methods of detecting temperatures based on NMR techniques are disclosed in EP 122000.

Magnetic resonance in Medicine 26, 308 (1992); EP-A-0 122 000; The Br. J. Radiology 64, 923 (1991); Eur. J. Vasc. Surg. 5, 383 (1991); J. Chem. Phys. 97, 8934 (1992) generically refer to the field of in vivo NMR imaging and diagnostic but do not refer either to in vivo measurements or to the use of chemical shift reagent.

The choice of the suitable compound is based on the evaluation of different parameters such as relaxivity, toxicity, distribution in the human body, excretion and so on. Three important properties are needed to use a complex, i.e. Gd⁽³⁺⁾, as a potential MRI contrast agent. Firstly, a high thermodynamic stability (and possibly kinetic), that's to say a low tendency to release free Gd⁽³⁺⁾ ion, highly toxic in vivo. Secondly, the presence of at least one water molecule directly coordinated to a metal in the inner coordination sphere and able to rapidly exchange with the bulk one. Thirdly, a high water solubility (≥0.5 M).

The paramagnetic complexes, which up to now have been preferably used as contrast agents in MRI medical diagnosis, are chelated complexes of aminopolycarboxylic acids and/or their derivatives or analogues, with Mn⁽²⁺⁾, Fe⁽³⁺⁾ and Gd⁽³⁺⁾ ions, which are the most widely investigated since they better meet the above mentioned needs. The other paramagnetic metal ions are not so efficient as relaxation agents, due to their very short relaxation times. On the contrary, a characteristic NMR property of complexes of these ions is the very high chemical shift of the magnetically active nuclei in the ligand. This property is partly transferred to the resonance characteristic of chemical species which interact with these complexes. When used for this aim in high-resolution spectroscopy, these complexes are called shift reagents (SR). Introduced during the '70s, basically as Eu⁽³⁺⁾ and Yb⁽³⁺⁾ complexes, they played a relevant role in the definition of organic molecule structures in solution since they produce a shift of nearby nuclei resonance values, and therefore a consequent easier reading of spectra data.
Fluorinated compounds can represent an alternative approach to the diagnostic imaging through magnetic resonance in comparison to the a.m. Gd⁽³⁺⁾ ,Mn⁽²⁺⁾ and Fe⁽³⁺⁾ paramagnetic compounds.

From the theoretical point of view, fluorinated substances used for the diagnostic imaging have some advantages: a) the absence, in fluorine spectral range, of signals coming from endogenous substances (fluorine is practically absent in biological systems); b) natural abundance (100%) and nuclear spin of ¹⁹F (I-1/2). But these advantages are heavily limited by an important factor: the quite long relaxation times of fluorine nucleus, which causes a severe reduction of signal/noise ratio (S/N) of the obtainable image and/or a remarkable delay of analysis times.

Thanks to its good biocompatibility properties, PFOB (perfluorooctylbromide, currently under investigation as potential blood substitute) is the most investigated product. This substance, however, has some other drawbacks due to:
a) multiplicity of ¹⁹F resonances generating a high signal dispersion;
b) excretion difficulties.

Other compounds which try to overcome the above mentioned drawbacks have been prepared. For instance, PFBD (perfluoro-2,2,2',2'-tetramethyl-4,4'-bis(1,3-dioxolane) (C₁₀F₁₈O₄) has been prepared, having the following formula: 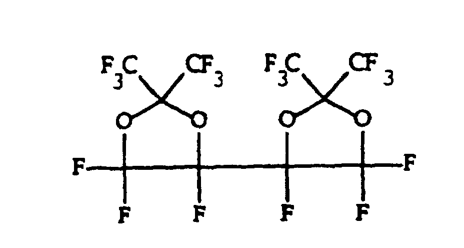 [Magn. Reson. Med., 20, 188 (1993)]

Another example comes from perfluoro-15-crown-5, constituted by 20 F, 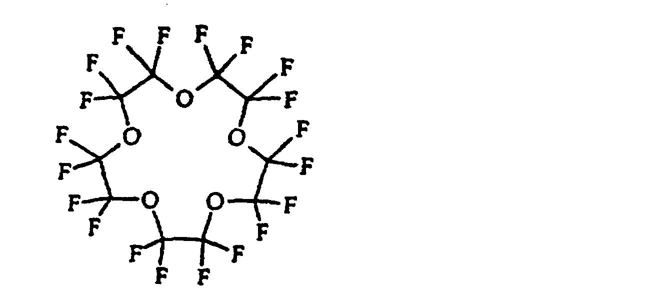 claimed as MRI contrast agent in patent EP 307863.

Some of these fluorinated compounds have been proposed as diagnostic probes in MR spectroscopy (MRS). In short, they are compounds containing at least two different kinds of fluorine atoms, whose different resonancesl can be used as chemical sensors of biological parameters, such as pH, pO₂, pCO₂, T, or more generally of the chemico-physical environment of organs or tissues. For instance patent applications EP-A-447013 and AU 633850 claim fluorinated compounds of the following type: 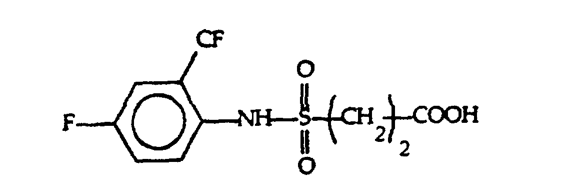 [Book of Abstracts, XI^{th} Meeting SMRM, p.3413]
to be used as sensors for measuring the pH in living tissues.

We have now unexpectedly found out, and this is one of the most remarkable aspect of this invention, that this type of application can be also performed in proton NMR spectroscopy, by using paramagnetic complexes endowed with shift reagent properties.

In addition, and this is another aspect of this invention, if in the paramagnetic metal complex, there are protons characterized by sufficiently intense signals and chemical shifts outside the interval of proton signal of nearby water and of the biological system constituents, the signal difference between bulk water and said paramagnetic complex protons can be exploited to obtain, thanks to Chemical Shift Imaging (CSI) technique, excellent images for the distribution of the contrast medium in the tissue or the organ under exam. Therefore, in contradiction to the universally accepted teaching of the prior art, said images comes from the proton resonance of the chelating or complexing agent and not from those belonging to water of the system under exam.

As far as MRS is concerned, these complexes are used as probes to measure biological parameters such as for instance pH, pO₂, pCO₂, T, in the tissues where the contrast medium preferentially concentrates. These applications, even if similar to those proposed for fluorinated derivatives, do not present the drawbacks belonging to these last ones, thus representing an unexpected and a remarkable technical improvement to the state of the art.

A remarkable advantage given by the use of the above mentioned compounds in Chemical Shift Imaging, resides in the extremely short T₁ values of paramagnetic compounds resonances which allow very rapid pulsations. This allows a high S/N ratio of the obtainable image, and the possibility of operating at low concentrations of contrast agent, with acceptable analysis times.

As a matter of example, if we use a complex of a lanthanum ion with the usual administered dosis in MRI, 0.1 mmol/kg, and we hypothesize an entirely intravascular distribution, we can reach a concentration of 1.5 mM, which generates a spectrum showing a good S/N ratio in few minutes (2-6), obtaining images showing a good distribution of the contrast agent.

As far as the application of such complexes in MRS are concerned, they can be properly used as probes to measure the temperature of the districts under exam. As a matter of fact it is known that the paramagnetic shift heavily depends from temperature, through the contact factor (Curie's behaviour, 1/T² dependent), and through the dipolar factor (1/T dependent).

Through suitable functionalizations, the paramagnetic shift can be made dependent from pH or from specific interactions with the substrates presenting the organs or tissues under exam.

This invention refers to a method of use of proton signals of paramagnetic metal-ion complexes in MRI, thanks to the use of Chemical Shift Imaging techniques, as well as in MRS for the control and recording of biological parameters.

A particularly suitable example which highlights the originality of this invention and its high potential diagnostic application is the Yb (III) complex of [αR*(αR*,α'R*,α''R*,α'''R*)]-(α,α',α'',α''')-tetramethyl-1,4,7,10-tetraazacyclododecan-1,4,7,10-tetraacetic acid (DOTMA) as hereunder represented. This product is cited in one article only (Brittain, H. G.; Desreux, J. F., Inorg. Chem., **1984**, 23, 4459), without mentioning its preparation and chemico-physical characteristics, apart from ¹H-RMN spectrum and a study concerning conformational isomers in solution.

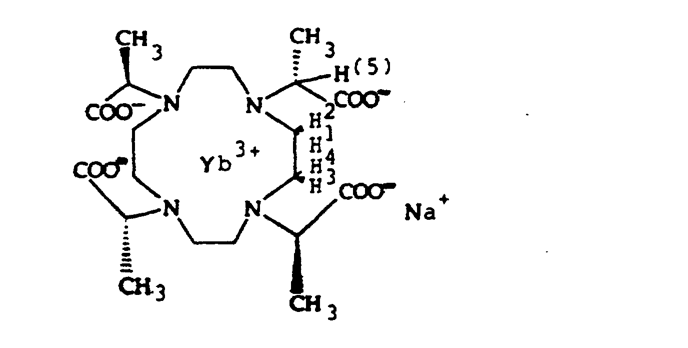

As far as the ligand type is concerned, that's to say DOTMA, it must be cited one preparation method published on Inorg. Chem., 32, 2912, **1993**, authors Tweedle M. F. et ai., starting from [αR*(αR*,α'R*,α''R*,α'''R*)]-(α,α',α'',α''')-tetramethyl-1,4,7,10-tetraazacyclododecan-1,4,7,10-tetraacetic acid, tetrabenzyl ester (DOTMA-TBE).

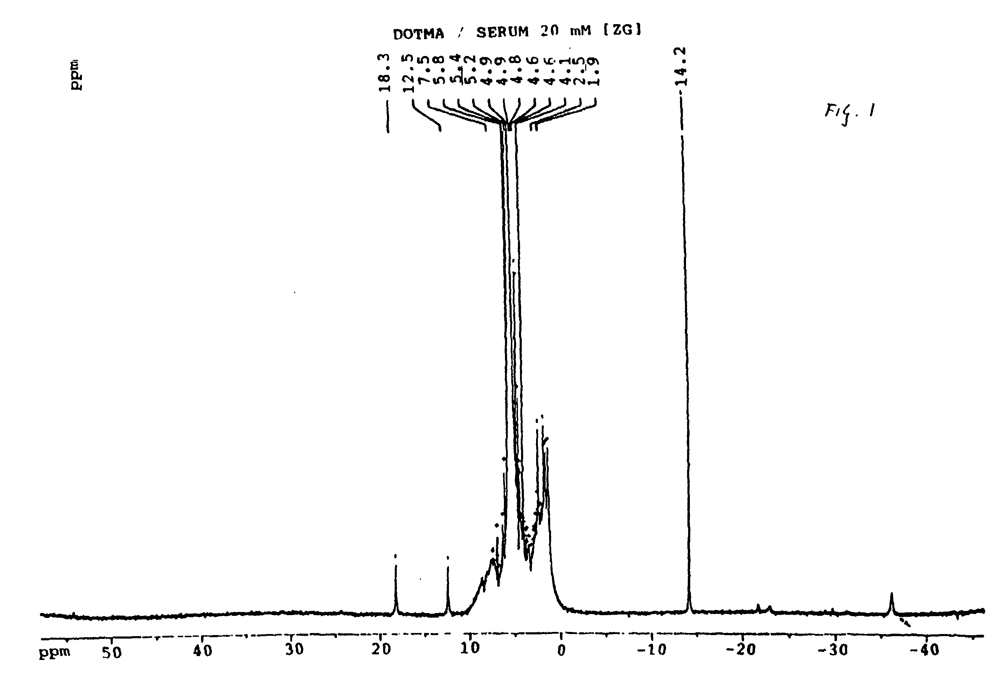

The presence of 12 equivalent protons belonging to the four -CH₃ groups, originates an intense unitary signal at about -14.2 ppm under the described conditions of Fig. 1, Example 3, providing a good S/N ratio in MRI exams.

In this case, the metal outdistances the signal of the four methyls of the propionic chain from water protons by about 20 ppm, so that the hydrogen nuclei of these methyls are made "different" from the hydrogen nuclei of bulk water, making this difference exploitable with CSI.

The same complex can be used in MRS techniques as probe for determining for instance the temperature of the district where the complex accumulates. For instance the chemical shift difference among non-equivalent protons such as H(1) and H(5) is dependent from the temperature of biological districts under exam. Therefore very interesting and useful information can be gathered by comparing the different proton resonances, thanks to the use of this technique.

Suitable paramagnetic complexes of this invention are substantially constituted by: a) a paramagnetic metal ion with atomic number selected between 21 and 29, 42, 44 and between 58 and 83 except for 64 and 71; b) a chelating agent selected from [αR*(αR*, α'R*, α''R*, α'''R*)]-(α,α',α'',α''')-tetramethyl-1,4,7,10-tetraazacyclododecan-1,4,7,10-tetraacetic acid, bis[tris(l-pyrazoil)boric acid, tris-N,N-dithiocarbamic acid and their salts having chemical shifts different from those of nearby water and being outside the range of the proton signals of the constituents of biological systems (said interval is usually between 0 and 10 ppm).

Of course, these paramagnetic complexes can be neutralized with organic and/or inorganic physiologically compatible bases or acids. Particularly preferred metal ion are V³⁺, Mn³⁺, Fe²⁺, Fe³⁺, Co⁺, Co²⁺, Ni²⁺, Mo³⁺, Ru³⁺, W³⁺, Re³⁺, Os³⁺, Yb³⁺, Dy³⁺.

In addition complexes with a balance between low and high spin shapes must be included. Due to the large differences in the NMR parameters of these two shapes, these systems can experience a wide chemical shift variation of their resonances according to the different environmental conditions. Suitable substituents can be introduced on these complexes to enable the transfer of environmental parameter variations (T, pO₂, pH,....) to the concentration variation of balanced shapes.

### EXAMPLE 1

Yb³⁺ complex with [1R-(1R*,4R*,7R*,10R*)](α,α',α'',α''')-tetramethyl-1,4,7,10-tetraazacyclododecan-1,4,7,10-tetraacetic acid sodium salt.

### A) [αR*(αR*,α'R*,α''R*,α'''R*)]-(α,α',α'',α''')tetramethyl-1,4,7,10-tetraazacyclododecan-1,4,7,-10-tetraacetic acid.

A solution of 43.2 g of (S)-2-chloropropionic acid (prepared according to Fu, S. C., J. Am. Chem. Soc., **1954,** 76, 6054) (0.40 mol) in 60 ml of water, is cooled to 5°C. and then the pH is adjusted to 5 with 20% NaOH without exceeding 5°C. 13.8 g of 1,4,7,10-tetraazacyclododecane (prepared according to Atkins T. J. et al., Org. Synth., **1978**, 58, 86) are added in portions and the solution is heated to 70°C for 24h, by keeping the pH at 10 by addition of 20% NaOH. After filtration of the solid, the solution is concentrated and to the residue are added further 10 g of (S)-2-chloropropionic acid (0.09 mol). Then it is retaken to the previous conditions (pH 10, 70°C) for additional 15 h, obtaining another portion of precipitate which is filtered. The two resulting precipitates are collected and dissolved in 1 l of water and the solution is acidified at pH 4 (through 37% HCl) and then electrodialyzed. The resulting solution is concentrated and the residue is diluted with cold EtOH. After filtration, 10.2 g (0.022 mol) of the desired compound are obtained.

| | | | |
|---|---|---|---|
| Yield | 28% | m.p. | > 250°C |
| K.F. | 0.30% (w/w) | | |
| HPLC | 98.2% (% area) | | |
| 0.1N NaOH | 99.0% (w/w) | | |

| Elemental Analysis | C | H | N |
|---|---|---|---|
| % calc. | 52.16 | 7.88 | 12.17 |
| % found | 51.91 | 7.90 | 12.12 |

¹H-RMN, ¹³C-RMN, IR and MS spectra are consistent with the indicated structure.

### B) Yb³⁺ complex of [αR*(αR*,α'R*,α''R*,α'''R*)]-(α,α',α'',α''')-tetramethyl-1,4,7,10-tetraazacyclododecan-1,4,7,10-tetraacetic acid sodium salt

2.3 g of [αR*(αR*,α'R*,α''R*,α'''R*)]-(α,α',α'',α''')-tetramethyl-1,4,7,10-tetraazacyclododecane-1,4,7,10-tetraacetic acid (0.005 mol) are suspended in 20 ml of water and solubilized at pH 6.5 by addition of 6 ml of 2N NaOH (0.012 mol). To the resulting solution, 1.94 g of YbCl₃·6H₂O (marketed product) (0.005 mol) are added in 10 ml of water by checking that the pH solution drops down to about 4. 2N NaOH is dropwise added to the mixture up to pH 5. The solution is kept at a constant pH (pH 6.5) by addition of 4ml of 2N NaOH (0.008 mol) during 10 h. After filtration and electrodialysis, the solution is taken to pH 6.5 through 1N NaOH and concentrated to dryness. 1.65 g (0.00253 mol) of the desired compound are obtained.

| | | | |
|---|---|---|---|
| Yield | 51% | m.p. | >250°C |
| K.F. | 2.89% (w/w) | | |

| Elemental Analysis | C | H | N | Na | Yb | Cl |
|---|---|---|---|---|---|---|
| % calc. | 36.81 | 4.94 | 8.59 | 3.52 | 26.52 | |
| % found | 36.05 | 5.15 | 8.49 | 3.02 | 25.18 | <0.10 |

¹H-RMN, ¹³C-RMN, IR and MS spectra are consistent with the indicated structure.

### EXAMPLE 2

Europium complex with [αR*(αR*,α'R*,α''R*,α'''R*)]-(α,α',α'',α''')-tetramethyl-1,4,7,10-tetraazacyclododecan-1,4,7,10-tetraacetic acid sodium salt.

In accordance with the procedure described in EXAMPLE 1, 2.3 g of [αR*(αR*, α'R*,α''R*,α'''R*)]-(α,α',α'',α''')-tetramethyl-1,4,7,10-tetraazacyclododecan-1,4,7,10-tetraacetic acid (0.005 mol) in 20 ml of water are reacted with 1.83 g of EuCl₃·6H₂O (marketed product) (0.005 mol) in 10 ml of water. 1.50 g of the desired compound are obtained (0.00237 mol).

| | | | |
|---|---|---|---|
| Yield | 47% | m.p. | >250°C (dec.) |
| K.F. | 3.06% (w/w) | | |

| Elemental Analysis | C | H | Eu | N | Na | Cl |
|---|---|---|---|---|---|---|
| % calc. | 38.04 | 5.11 | 24.06 | 8.87 | 3.64 | |
| % found | 37.00 | 5.69 | 23.26 | 8.63 | 3.04 | <0.10 |

¹H-RMN, ¹³C-RMN, IR and MS spectra are consistent with the indicated structure.

### EXAMPLE 3

Proton spectra recorded by AMS-400 spectrometer, ¹H 400,13 MHz; ¹³C 100.61 MHz; internal reference: H₂O 4.8 ppm.
Figure 1: proton spectrum of a 20mM solution of serum Yb-DOTMA SERONORM-HUMAN™ (NYCOMED).

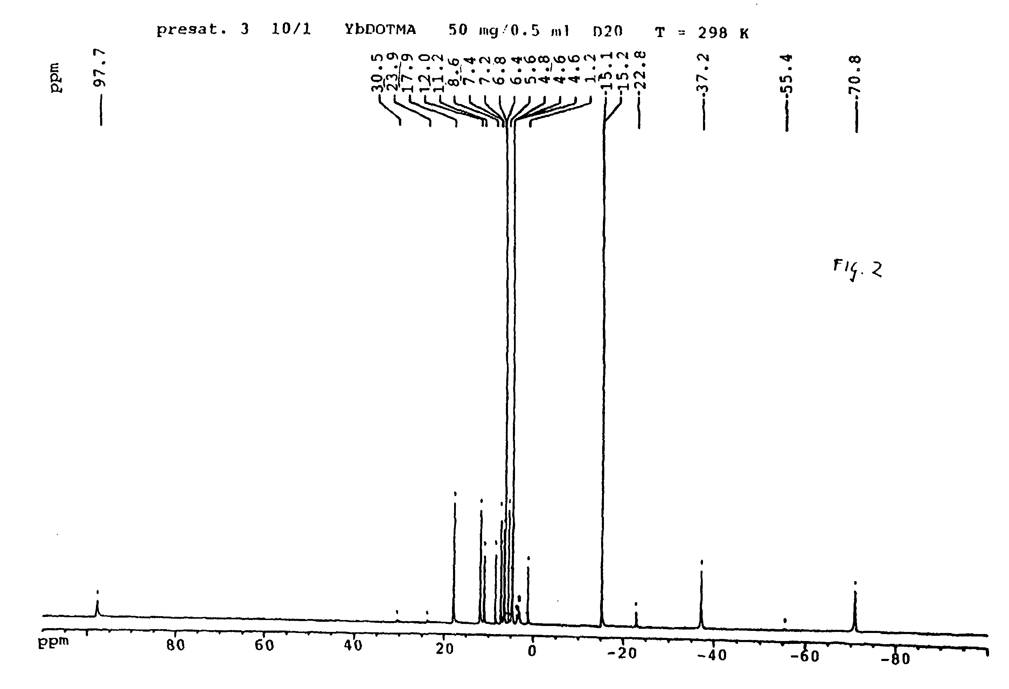

Figure 2: proton spectrum of a 0.12 M solution of Yb-DOTMA in 0.5 ml of D₂O.

## Claims

1. The use of a paramagnetic chelating agent complex wherein the paramagnetic metal ion is selected from those having atomic number between 21 and 29, 42, 44 and between 58 and 83 except for 64 and 71 and wherein the chelating agent is selected from [αR*(αR*, α'R*, α''R*, α'''R*)]-(α,α',α'',α''')-tetramethyl-1,4,7,10-tetraazacyclododecan-1,4,7,10-tetraacetic acid, bis-[tris(1-pyrazoil)boric acid, tris-N,N-dithiocarbamic acid and their salts, for the preparation of an MRI contrast agent for obtaining images of organs, tissues, biological districts of human or animal body through nuclear magnetic resonance (NMR), by chemical shift imaging techniques.

2. The use according to claim 1 wherein the paramagnetic metal ion is selected from V³⁺, Mn³⁺, Fe²⁺, Fe³⁺, Co⁺, Co²⁺, Ni²⁺, Mo³⁺, Ru³⁺, W³⁺, Re³⁺, Os³⁺, Yb³⁺, Dy³⁺.

3. The use according to claim 1 or 2 wherein the paramagnetic chelate/complex is salified with physiologically tolerable organic and/or inorganic bases or acids.

## Patentansprüche

1. Verwendung eines paramagnetischen Chelatbildnerkomplexes, wobei das paramagnetische Metallion ausgewählt wird unter solchen mit einer Atomzahl zwischen 21 und 29, 42, 44 und zwischen 58 und 83, ausgenommen von 64 und 71, und wobei der Chelatbildner ausgewählt wird aus [αR*(αR*; α'R*, α''R*, α'''R*)]-(α,α',α'',α''')-tetramethyl-1,4,7,10-tetraazacyclododecan-1,4,7,10-tetraessigsäure, Bis-[tris-(1-pyrazoil)borsäure, Tris-N,N-dithiocarbaminsäure und ihren Salzen zur Herstellung eines MRI-Kontrastmittels zur Herstellung von Bildern von Organen, Geweben, biologischen Gebieten des menschlichen oder tierischen Körpers durch kernmagnetische Resonanz (NMR) gemäß chemischen Verschiebungsverfahren.

2. Verwendung nach Anspruch 1, wobei das paramagnetische Metallion ausgewählt wird aus V³⁺, Mn³⁺, Fe²⁺, Fe³⁺, Co⁺, Co²⁺, Ni²⁺, Mo³⁺, Ru³⁺, W³⁺, Re³⁺, Os³⁺, Yb³⁺, Dy³⁺

3. Verwendung nach Anspruch 1 oder 2, wobei das/der paramagnetische Chelat/Komplex mit physiologisch tolerierbaren organischen und/oder anorganischen Basen oder Säuren in Salzform überführt ist.

## Revendications

1. L'utilisation d'un complexe paramagnétique d'agent chélateur, dans lequel l'ion métallique paramagnétique est choisi parmi ceux qui ont un numéro atomique de 21 à 29, 42, 44 et de 58 à 83 à l'exception de 64 et 71, et dans lequel l'agent chélateur est choisi parmi l'acide [αR*(αR*,α'R*,α"R*,α'''R*)]-(α, α', α",α''')-tétraméthyl-1,4,7,10-tétraazacyclododécane-1,4,7,10-tétraacétique, l'acide bis[tris(1-pyrazoyl)borique, l'acide tris-N,N-dithiocarbamique et leurs sels, pour la préparation d'un agent de contraste pour IRM pour l'obtention d'images d'organes, tissus, régions biologiques d'un corps humain ou animal au moyen de la résonance magnétique nucléaire (RMN), par des techniques d'imagerie par déplacement chimique.

2. L'utilisation selon la revendication 1, dans laquelle l'ion métallique paramagnétique est choisi parmi V³⁺, Mn³⁺, Fe²⁺, Fe³⁺, Co⁺, Co²⁺, Ni²⁺, Mo³⁺, Ru³⁺, W³⁺, Re³⁺, Os³⁺, Yb³⁺, Dy³⁺.

3. L'utilisation selon la revendication 1 ou 2, dans laquelle le chélate/complexe paramagnétique est salifié par des bases ou acides organiques et/ou minéraux physiologiquement tolérables.
